# EUROPEAN PATENT APPLICATION

(11) **EP 4 851 977 A1**
(43) Date of publication of application: **09.12.2026**
(21) Application number: 24920759.8
(22) Date of filing: 08.04.2024
(51) Int. Cl.: G09B 23/30, G09B 23/28

(54) **HUMANOID HEMODIALYSIS SIMULATOR**

(30) Priority: 30.01.2024 BR 102024001914
(71) Applicant: Silva, Renata Aparecida Candido Da, 86601-006 Rolândia/PR (BR)
(72) Inventor: Silva, Renata Aparecida Candido Da, 86601-006 Rolândia/PR (BR)
(74) Representative: Torner, Juncosa I Associats, SL
(86) International application number: PCT/BR2024/050143
(87) International publication number: WO 2025/160638

(57) **Abstract**

The present invention relates to the technical field of simulators for general medical and hospital use and the basic principle thereof is to provide a humanoid hemodialysis simulator (l) having its own specific electromechanical structure based on the incorporation of a suction pump (2) having an activation circuit, a central reservoir (3) with supports and connections, a rear inlet (4), a rear protective door (5), and a flow control drive circuit (6), with the aim of fully optimizing the structural and operational procedures for pumping blood between a standard hemodialysis machine and the hemodialysis simulator (1), specifically relating to the "Simulator for hemodialysis process" disclosed in patent application BR 10 2020 003433 2, filed on 19 February 2020 and published on 31 August 2021, of the same applicant and inventor.

## Description

The present patent is related to the technical field of simulators for medical and hospital use in general, more specifically to a humanoid hemodialysis simulator that, according to its general characteristics, provides, as its basic principle, the formation of a hemodialysis simulator in its own specific electromechanical humanoid structure based on the installation of a suction pump with an activation circuit, a central reservoir with mounts and connections, a rear protective door, a rear inlet, and a flow control drive circuit, with a view to fully optimize, in an extremely practical, safe and economic way, the procedures for setting up and administering blood flow between a standard hemodialysis machine and the hemodialysis simulator, specifically the "Simulator for the Hemodialysis Process", object of the patent application BR 10 2020 003433 2, filed on February 19, 2020, and published on August 31, 2021, with the same owner and inventor, Renata Aparecida Candido da Silva and having as a basis a hemodialysis simulator with great strength, safety and versatility.

With specific design and shape and easy access for better adaptation and user safety, practicality in handling and functionality, very affordable costs, and, due to its general characteristics and dimensions, easily adaptable to a wide range of humanoids, hemodialysis machines, locations, patients and nursing technicians in general, regardless of the characteristics that they can present.

It should be noted that hemodialysis is a procedure in which a specialized machine filters and cleans all of the kidney patient's blood, that is, performing part of the work that the diseased kidney cannot carry out to the desired and necessary standard. The process involves removing harmful waste products, such as excess salt and fluids, from the patient's body, as well as controlling blood pressure and helping the body maintain the balance of substances such as sodium, potassium, urea, and creatine.

Hemodialysis is indicated for patients with severe acute or chronic kidney failure. Therefore, the decision to initiate this treatment is made exclusively by a nephrologist, i.e., a physician specializing in kidney diseases. It should be noted that dialysis is not intended to treat kidney disease but rather to replace the function of kidneys that are no longer working properly.

Treatment for kidney failure may begin with specific medications designed to control symptoms and stabilize the conditions; however, in cases where medication is insufficient and the disease progresses, it may be necessary to start hemodialysis, so this decision is made in consultation with the patient and their nephrologist. Hemodialysis sessions are usually performed in specialized clinics or hospitals by nurses, specialist nursing technicians, and other healthcare professionals.

Dialysis generally involves filtering the blood, which is gradually drawn from the body through a special needle inserted into an arteriovenous fistula (AVF) - a connection between a small artery and a small vein that is created to make the vein thicker and more resistant, allowing punctures to be performed without complications. The fistula can be created using either the patient's own veins or synthetic materials, and this is done via a minor surgical procedure on the arm or leg performed by a vascular surgeon under local anesthesia.

Similarly, dialysis can also be performed using a catheter inserted directly into a vein in the kidney patient's neck (subclavian or right or left jugular), chest, or groin under local anesthesia, the catheter being generally a temporary option for kidney patients who need dialysis but do not yet have a fistula. The main problems associated with catheter use are blockages and infections resulting from improper handling, which often requires its removal and the implantation of a new catheter so that sessions can continue.

It should be noted that hemodialysis is a therapeutic modality intended for the treatment of critically ill and/or severely ill patients; therefore, it is the responsibility of the nephrologist nursing teams to perform technical procedures related to the direct and continuous care of kidney patients undergoing hemodialysis at every stage of the process. This care includes preparation, fistula puncture or catheter management in kidney patients, monitoring, programming the hemodialysis machine and setting up the circuit, changing solutions, and providing physical and emotional care to kidney patients, among other tasks.

However, the high level of complexity and, consequently, the significant challenges involved in training nephrology nursing teams to a high standard, particularly for hemodialysis procedures must be acknowledged. These procedures are all based on traditional "at-the-machine" training conducted during the patients' hemodialysis sessions, using the time allotted for their treatment and thereby reducing the opportunity for optimal removal of waste products from the patient's body.

Specifically, patients with chronic kidney disease depend exclusively on a hemodialysis machine to remove toxins from their body because they have lost kidney function. Therefore, every time a professional shuts down the hemodialysis machine to train someone else, they are directly violating the patient's right to receive their treatment within the prescribed timeframe and according to the established protocols - in other words, it would be as if the professional were taking the patient's medication.

Thus, all questions regarding the technical procedures that must be performed are asked directly in front of patients undergoing hemodialysis, that is, they are naturally in a delicate situation and end up feeling completely insecure about the application and learning process, which makes it difficult for nephrology nursing teams and leads to significant exposure for both parties.

A comprehensive review of the literature to determine the current state of the art regarding hemodialysis simulators, primarily simulators in humanoid form, object of the present patent of invention, only the patent application BR 10 2020 003433 2 - "Simulator for the Hemodialysis Process", was found as relevant to the state of the art related to the specific object claimed.

However, although this initial patent application demonstrated excellent results in terms of the learning outcomes for nephrology nursing teams, having become a disruptive technology - or even a disruptive innovation - that brought about a complete break with the medical standards, models, or technologies already established in hemodialysis procedures, the simulator described herein still has some drawbacks that need to be significantly addressed through improvements and optimizations to its basic structure, such as, for example, the procedure for pumping blood flow within it as recirculation and the procedures for filling, maintaining, and accessing/securing the blood reservoir.

Given this scenario, it has become essential for nephrology nursing teams who are currently undergoing training, the development of a new, versatile, safe and practical hemodialysis simulation structure based on a set of improvements and optimizations to the structure already described in the prior patent application "Simulator for the Hemodialysis Process" that is capable of fully overcoming these significant existing drawbacks, reducing the cost and duration of training, as well as the potential suffering of kidney patients undergoing hemodialysis.

Thus, the patent in question is characterized by combining components and processes in a differentiated design to meet the various requirements the nature of its use demands, that is, the simulation of hemodialysis procedures in patients with severe acute or chronic kidney failure. This design guarantees a hemodialysis simulator of great efficiency, functionality, resistance, durability, safety, versatility, practicality, accuracy and cost-effectiveness due to the excellent technical qualities added, which provide advantages and improvements in the set of training procedures for nephrology nursing teams by using a humanoid simulator and, whose general characteristics differ from other forms and models known in the current state of the art.

The present patent consists on the use of a modern, efficient, safe and functional humanoid hemodialysis simulator comprising a set of electromechanical and medical solutions that are properly incorporated, forming a complete and unique hemodialysis simulator with exclusive design, beautiful aesthetics and unique characteristics, incorporating its own specific electromechanical humanoid structure, of high durability and resistance, and containing a perfectly integrated and symmetrically arranged suction pump with an activation circuit, as a component for distributing blood flow within the hemodialysis simulator from the central reservoir; a central reservoir with mounts and connections as a component for storing blood inside the hemodialysis simulator for subsequent distribution; a rear protective door as a component for protecting the internal compartment of the hemodialysis simulator and controlling the access to the central reservoir; a rear inlet as a component for external blood supply to the central reservoir via the upper tube; and a flow control drive circuit as a component for regulating blood flow within the hemodialysis system, thereby enabling the creation of a single, complete, and safe unit whose internal and external shapes and configurations allow for perfect adaption to a wide range of patients and hemodialysis procedures in general, having been specifically designed for these purposes.

Thus, the general design of the present humanoid hemodialysis simulator, object of the present patent, is based entirely on its simple, robust structuring with a minimum of components and extremely simplified, safe and optimized operation that are also very practical to manufacture and maintain, in order to generate a practical and efficient hemodialysis simulator based on improvements and optimizations introduced in a humanoid simulator with the purpose of humanizing and simplifying the training of nephrology nursing teams who perform hemodialysis procedures, that is, a humanoid in the form a doll or any other format with an improved and optimized set of components that allow for the full simulation of blood flow like that of the hemodialysis procedure in patients with kidney disease, as kidney patients are simulated at all stages of the hemodialysis procedure, from preparation through to discharge.

More specifically, inside the hemodialysis simulator, which is connected to a hemodialysis machine, the patient is replaced by a humanoid, and the entire circulatory system of a hemodialysis procedure is simulated, providing a perfectly realistic hemodialysis session, in which the blood reservoir, the reservoir suction pump, and the arterial and venous lines extending from the upper limbs, subclavian, right or left jugular veins, as well as the abdomen are connected.

These new components, integrated directly into the hemodialysis simulator, significantly improve - through their comprehensive optimization, the entire process of learning, training and practicing hemodialysis by nephrology nursing teams, from operating the hemodialysis machine to preparing and disconnecting the kidney patient undergoing treatment, completely replacing this kidney patient with the simulator and hemodialysis for the training of new nursing teams or any healthcare professional.

The present hemodialysis simulator is based on the application of components and processes in a differentiated design, without, however, achieving a high degree of sophistication or complexity, making it possible to solve some of the main drawbacks of the other shapes and models known in the current state of the art and used in the hemodialysis simulation procedures in kidney patients with the purpose of improving the learning of nursing teams, that operate in a working range in which traditional forms and/or models have difficulties in use and application, low efficiency and performance, cause frequent accidents or deterioration, risk of iatrogenic complications, low versatility, low accuracy, generating insecurity and low performance, high losses, high cost, complex handling, high maintenance, significant time waste, and complex manufacturing.

The objects, advantages, and other important characteristics are more easily understood when read together with the attached figures, in which:

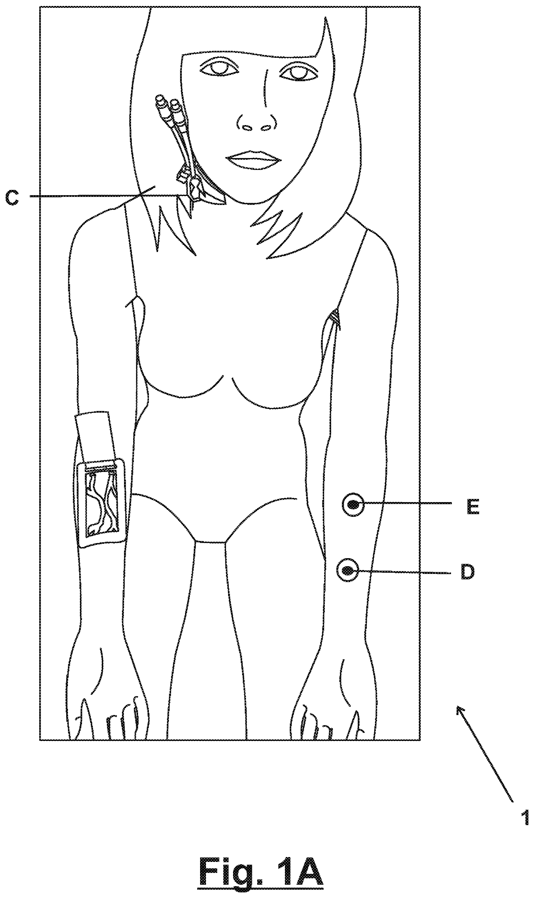

Figure 1 is a perspective view of the humanoid hemodialysis simulator.

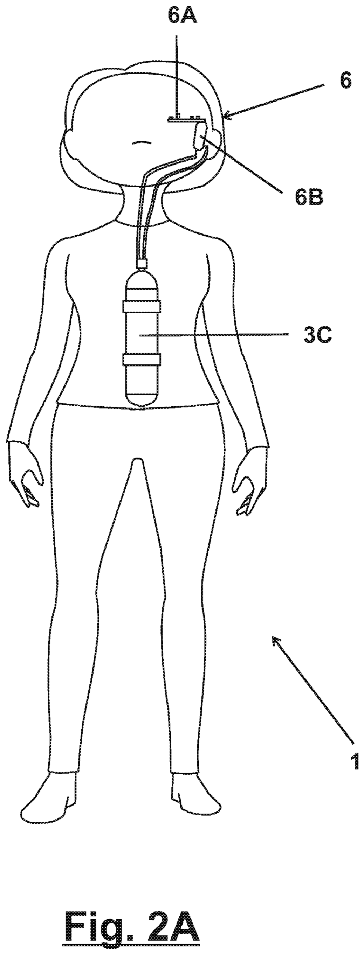

Figure 2A is a schematic view of the flow control drive circuit of the humanoid hemodialysis simulator.

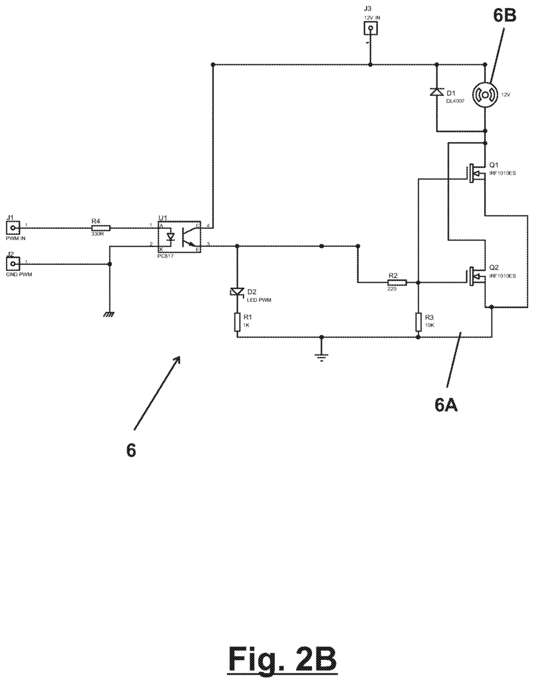

Figure 2B is a wiring diagram for the flow control drive circuit of the humanoid hemodialysis simulator.

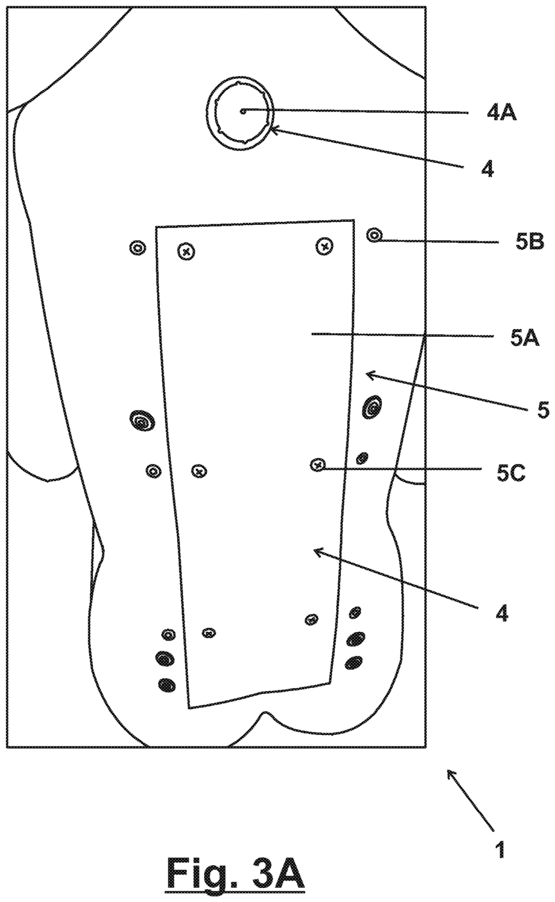

Figure 3A is a rear perspective view of the humanoid hemodialysis simulator showing the rear protective door and the rear inlet in detail.

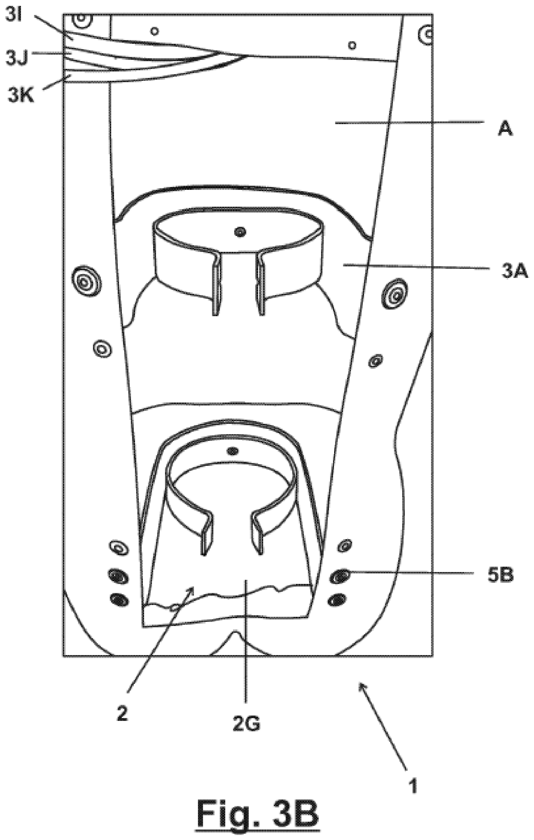

Figure 3B is a rear perspective view of the humanoid hemodialysis simulator showing the internal compartment with the central reservoir mount and the suction pump in detail.

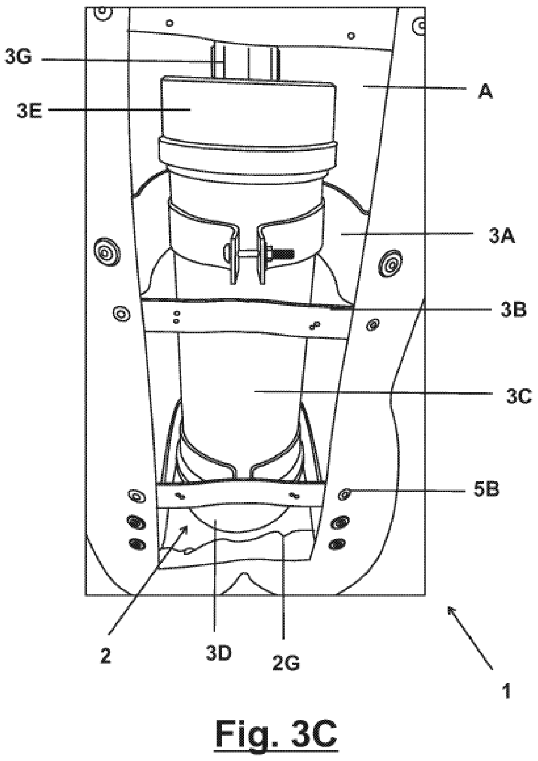

Figure 3C is a rear perspective view of the humanoid hemodialysis simulator showing the internal compartment with the central reservoir, its mount, and the suction pump in detail.

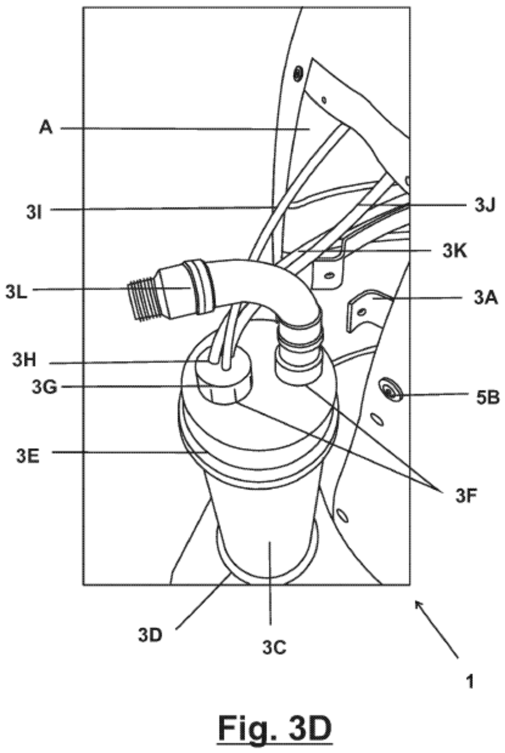

Figure 3D is a rear perspective view of the humanoid hemodialysis simulator showing the central reservoir and its connections located outside the internal compartment.

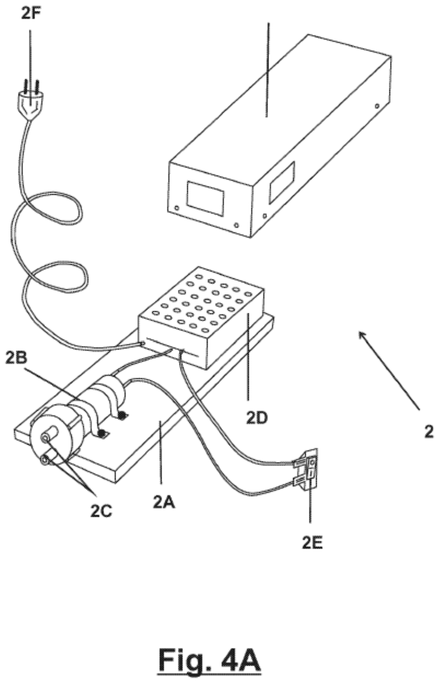

Figure 4A is a perspective view of the suction pump of the humanoid hemodialysis simulator.

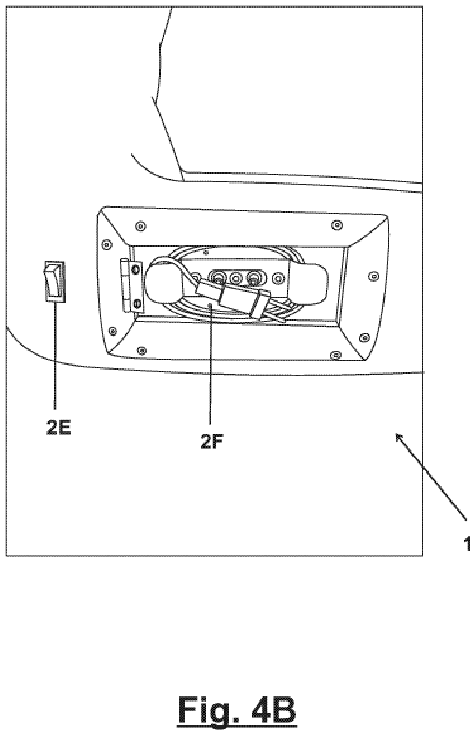

Figure 4B is a perspective view of the suction pump on/off switch of the humanoid hemodialysis simulator located on the inner thigh.

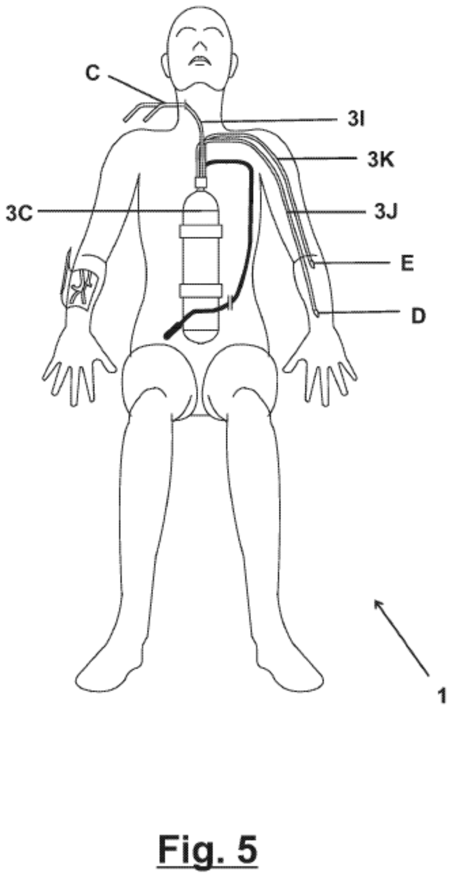

Figure 5 is a schematic view of the humanoid hemodialysis simulator without the flow control drive circuit.

As can be inferred from the attached figures, which illustrate and integrate this descriptive report of the patent for "Humanoid Hemodialysis Simulator", Figure (1A) provides a general overview of the hemodialysis simulator, comprising a complete hemodialysis simulator (1) which has its own characteristics and incorporates its own specific electromechanical humanoid structure, internal and external configurations that adapt to a wide range of humanoids and hemodialysis machines in general, and containing perfectly integrated and symmetrically arranged
- a suction pump (2) arranged horizontally and symmetrically centered along the entire length of the inner base of the internal compartment (A), in the inner rear portion of the hemodialysis system (1), and symmetrically centered beneath the lower portion of the central reservoir (3), wherein the suction pump (2) comprises a base (2A) arranged horizontally and symmetrically along the entire lower length of the suction pump (2); a water vacuum pump (2B) arranged horizontally and symmetrically at one end of the base (2A) and connected via its two inlet and outlet connections (2C) to the central reservoir (3); a switched-mode stabilizer (2D) arranged horizontally and symmetrically at the other end of the base (2A) and connected to the water vacuum pump (2B); an on/off switch (2E) arranged symmetrically on the outside of the internal compartment (B) on the thigh of the hemodialysis simulator (1) and connected to the water vacuum pump (2B) and the switched-mode stabilizer (2D); a power cable (2F) arranged symmetrically inside an internal compartment on the thigh of the hemodialysis simulator (1) and connected to the switched-mode stabilizer (2D); a protective cover (2G) arranged horizontally and symmetrically over and along the entire length of the suction pump (2); and an activation circuit with a microcontroller located inside the protective cover and connected to the water vacuum pump (2B);
- a central reservoir (3) arranged vertically and symmetrically centered inside the internal compartment (A) and above the pump and suction unit (2), within the inside of the hemodialysis system (1) chest, and comprising two curved rear mounts/clamps (3A) arranged horizontally, parallel and symmetrically spaced at the rear portion of the internal compartment (A); two straight front locks (3B) arranged horizontally, parallel and symmetrically spaced in the front part of the internal compartment (A); a cylindrical reservoir (3C) arranged vertically and symmetrically supported on the vacuum pump (2), fitted/secured by its rear faces around the front faces of the two rear mounts (3A), and locked by its front faces against the rear faces of the two front locks (3B), serving as storage for all the blood necessary for the hemodialysis simulator's blood flow (1); a cylindrical lower cover (3D) arranged horizontally, parallel and symmetrically beneath and around the entire length of the lower face of the reservoir (3C); a cylindrical top cover (3E) arranged horizontally, parallel and symmetrically beneath and around the entire length of the lower surface of the reservoir (3C) and having two circular upper threaded openings (3F) arranged symmetrically spaced on the top cover (3E); a cylindrical threaded overcap (3G) arranged symmetrically around one of the upper openings (3F) and having a sealing ring arranged around the entire upper length of the inner part of the threaded overcap (3G) and three circular upper openings (3H) arranged symmetrically spaced on the threaded overcap (3G) and connected by a tube/connector (3I) originating from the insertion point of the subclavian catheter in the neck (C), by a tube/connector (3J) originating from the arterial fistula exit point of the arterial line in the left wrist region (D), and by a tube/connector (3K) originating from the venous return exit point of the venous line in the left wrist region (E), to guarantee blood flow inside the hemodialysis simulator (1); and a curved tube (3L) arranged symmetrically connecting the other upper opening (3F) to the rear inlet opening (4), serving as the inlet and outlet for the fluid that flows into the reservoir;
- the rear inlet (4) that is circular and arranged parallel to and symmetrically centered on the rear upper part of hemodialysis system (1) chest, aligned with and spaced from the upper endo of the rear protective door (5), connected to the inside of the internal compartment (A) of the hemodialysis system (1) and to the reservoir (3C) via the tube (3L), and comprising a threaded cap (4A) arranged symmetrically around the entire outer perimeter of the rear inlet (4), serving as the inlet and outlet for all the artificial blood fluid that supplies the reservoir (3C) of the hemodialysis simulator (1);
- the rear protective door (5) arranged vertically, parallel to, and symmetrically centered on the rear of the hemodialysis system (1) chest, fully aligning with the opening of the internal compartment (A) of the hemodialysis simulator (1), comprising a plate (5A) arranged vertically, parallel to, and symmetrically along the entire length of the opening of the internal compartment (A); a set of fixed fasteners (5B) arranged symmetrically spaced around the entire length of the rear of the hemodialysis simulator (1) chest, adjacent to the rear protective door (5) and securing the structure of the rear protective door (5); and a set of removable fasteners (5C) arranged symmetrically spaced around the entire length of the lateral edges of the rear protective door (5) and securing/locking the rear protective door to the hemodialysis simulator (1); and
- a flow control drive circuit (6) arranged symmetrically within the cranial region of the hemodialysis simulator (1) and connected with the microcontroller-based activation circuit of the suction pump (2), and comprising an integrated circuit microcontrollers (6A) featuring Power (J1), ground (J2) and voltage input (J3); a DC power supply Battery; and an internal pump (6B) connected to the integrated circuit with microcontrollers (6A) and to the reservoir (3C), operating simultaneously with the water vacuum pump (2B) to assist in pumping fluid through the internal circuit of the humanoid simulator (1) and the hemodialysis machine.

The humanoid hemodialysis simulator, depending on the application requirements, may include an electrocardiogram (ECG) simulator located inside the hemodialysis simulator and connected to the flow control drive circuit, allowing the heartbeats of kidney patients to be simulated by evaluating the heart's electrical activity through electrodes attached to the skin, that is, also detecting the heart rate and the number of beats per minute.

The humanoid hemodialysis simulator, depending on the application requirements, may comprise a structure shaped like any pet rather than a humanoid form, since the procedures and equipment used for hemodialysis in pets, regardless of the species, are the same as those used in humans.

The humanoid hemodialysis simulator, depending on the application requirements, may consist of a frame with lower limbs made of foam material, allowing the legs to be flexible and bendable for storage in carry-on-style cases, ensuring safer and more convenient transport.

Inserting the suction pump (2) into the hemodialysis simulator (1) allows the suction pump (2) to generate an optimal pressure for pumping blood flow within the hemodialysis simulator (1) through its activation by the microcontroller-based activation circuit, whereby the microcontroller of the activation circuit emits a PWM (pulse width modulation) signal, intended to control the suction pump pressure (2).

The design of a new central reservoir (3) located within the internal compartment (A) in the simulator chest, along with its new seals and dedicated connections, is highly necessary due to the logistics, transportation and packaging procedures involved in delivering the hemodialysis system (1) to the treatment sites, since the old container was very heavy and, more importantly, the blood fluid inside it leaked frequently due to the fragility of the old seals and connections on the container lid, as well as for security against unauthorized access such as tampering.

The addition of a rear inlet (4) on the upper rear part of the humanoid's torso was necessary to allow a practical and safe manual procedure for filling and draining the central reservoir (3) without exposing the interior of the hemodialysis simulator (1). The previous version of the hemodialysis simulator required the old door to be fully open to perform manual filling and draining for the old reservoir.

The redesigned rear protective door (5) installed at the rear of the hemodialysis simulator (1) no longer features the padlock or the pair of hinges found in the previous version; these have been replaced by a set of fixed (5B) and movable (5C) fasteners arranged around the entire length of the plate (5A) of the rear protective door (5). This change was necessary due to the flimsy construction and opening mechanism of the previous version, as the sides of that older model were vulnerable when opened, since the padlock was only on one side of the door, while the pair of hinges on the other side could be easily removed.

Thus, with this modification to the rear protective door (5), which incorporates a combination of fixed (5B) and movable (5C) fasteners, the new rear protective door (5) offers greater safety, including the ability to accommodate the new central reservoir (3) practically and securely within the internal compartment (A), whereas previously the reservoir was completely unsecured inside the previous version of the humanoid simulator.

The primary purpose of incorporating a flow control drive circuit (6) into the hemodialysis simulator (1) is to drive a pump in order to maintain blood flow within the hemodialysis simulator (1) during hemodialysis procedures. Thus, the flow control drive circuit (6) and its components such as the integrated circuit with a microcontroller (6A) and the internal pump (6B), which are critical to the proper functioning of the hemodialysis simulator (1), are installed in the upper part of the hemodialysis simulator (1), specifically in the cranial region, so that its inlet and outlet are connected directly to the suction pump (2), as this enables the crucial and necessary process of blood flow recirculation.

It should be noted that the humanoid hemodialysis simulator, according to its new internal technological development, can be used in both humans and a wide range of animals without any significant changes to its structure, components, or method of application and operation.

As the humanoid hemodialysis simulator has its components fully integrated with each other, nothing comes loose and has nothing to break or bend, a high level of performance and efficiency is achieved, combined with high durability and absolute safety. After being fully integrated with each other, and to the simulation humanoid, as well as to the hemodialysis machine, the components are cohesive, thus preventing them from coming loose on their own when in use, making the set fully available for the simulation procedures for hemodialysis processes in the training of nephrology nursing teams. In this way, the hemodialysis simulator (1) can be used without concerns of any kind, especially regarding the durability and safety of its components, in addition to the safety of nursing technicians and patients.

The humanoid hemodialysis simulator offers the following specific advantages: complete safety for kidney patients, as they no longer participate actively in training sessions for nursing teams or healthcare professionals in general, and are replaced by the hemodialysis simulator; high level of technical training among nephrology nursing teams, as they are now able to practicer without having to directly care for kidney patients, who are replaced by the hemodialysis simulator; a low rate of accidents caused by technical errors on the part of medical teams, as they can practice with greater flexibility, focus and regularity; and a high capacity to incorporate the wide variety of situations found in hemodialysis procedures into training sessions.

In view of all of the above, the medical simulator can be well received by nephrology nursing teams and chronic kidney patients in general, as the humanoid hemodialysis simulator has numerous advantages, such as: safety, reliability, and flexibility in application; good yield and performance in its application due to its general design; high comfort, convenience, and safety to nursing teams; exceptional strength and overall durability, combined to little or no wear on the set as a whole; affordable costs, enabling an excellent cost/benefit ratio; practical and safe for a wide range of nursing teams; very low and practical overall maintenance; high sterilization capacity; a perfect and direct adaptation to the most diverse types of hemodialysis machines; high operational accuracy; excellent versatility in regulating blood flow and volume; high ergonomics; and the certainty of having a hemodialysis simulator (1) that fully meets the current legislation and standards and the basic conditions necessary for its application in its entirety.

All these attributes allow to classify the humanoid hemodialysis simulator as a totally versatile, efficient, practical and safe means for carrying out simulation procedures in hemodialysis processes on the most diverse types of chronic kidney patients, with the purpose of providing a wide variety of training programs for a broad range of nephrology nursing teams in various settings such as hospitals and clinics, regardless of their general characteristics, being also very easy to apply and handle, combined with great performance and excellent general characteristics; however, it is not limited, at any moment, to the representations described herein and must be understood in the broadest sense, that is, it is not limited to the specific form revealed and other similar forms are also included within the claimed scope.

## Claims

1. "HUMANOID HEMODIALYSIS SIMULATOR", comprising a hemodialysis simulator **characterized by** incorporating, into its humanoid structure, integrated and arranged components:
- a suction pump (2) arranged horizontally and symmetrically centered along the entire length of the inner base of the inner compartment, in the inner rear portion of the hemodialysis system (1) chest, and symmetrically centered beneath the lower portion of the central reservoir (3), wherein the suction pump (2) comprises a base (2A) arranged horizontally and symmetrically along the entire lower length of the suction pump (2); a water vacuum pump (2B) arranged horizontally and symmetrically at one end of the base (2A) and connected via its two inlet and outlet connections (2C) to the central reservoir (3); a switched-mode stabilizer (2D) arranged horizontally and symmetrically at the other end of the base (2A) and connected to the water vacuum pump (2B); an on/off switch (2E) arranged symmetrically on the outside of the internal compartment (B) on the thigh of the hemodialysis simulator (1) and connected to the water vacuum pump (2B) and the switched-mode stabilizer (2D); a power cable (2F) arranged symmetrically inside an internal compartment on the thigh of the hemodialysis simulator (1) and connected to the switched-mode stabilizer (2D); a protective cover (2G) arranged horizontally and symmetrically over and along the entire length of the vacuum pump (2); and an activation circuit with a microcontroller located inside the protective cover and connected to the water vacuum pump (2B);
- a central reservoir (3) arranged vertically and symmetrically centered inside the internal compartment (A) and above the pump and suction unit (2), within the inside of the hemodialysis system (1) chest, and comprising two curved rear supports/clamps (3A) arranged horizontally, parallel and symmetrically spaced at the rear portion of the internal compartment (A); two straight front locks (3B) arranged horizontally, parallel and symmetrically spaced in the front part of the internal compartment (A); a cylindrical reservoir (3C) arranged vertically and symmetrically supported on the vacuum pump (2), fitted/secured by its rear faces around the front faces of the two rear supports (3A) and locked by its front faces against the rear faces of the two front locks (3B); a cylindrical lower cover (3D) arranged horizontally, parallel and symmetrically beneath and around the entire length of the lower face of the reservoir (3C); a cylindrical top cover (3E), arranged horizontally, parallel and symmetrically beneath and around the entire length of the lower surface of the reservoir (3C) and having two circular upper threaded openings (3F) arranged symmetrically spaced on the top cover (3E); a cylindrical threaded overcap (3G) arranged symmetrically around one of the upper openings (3F) and having a sealing ring arranged around the entire upper length of the inner part of the threaded overcap (3G) and three circular upper openings (3H) arranged symmetrically spaced on the threaded overcap (3G) and connected by a tube/connector (3I) originating from the insertion point of the subclavian catheter in the neck (C), by a tube/connector (3J) originating from the arterial fistula exit point of the arterial line in the left wrist region (D), and by a tube/connector (3K) originating from the venous return exit point of the venous line in the left wrist region (E); and a curved tube (3L) arranged symmetrically connecting the other upper opening (3F) to a rear inlet opening (4);
- the rear inlet opening (4) that is circular and arranged parallel to and symmetrically centered on the rear upper part of the hemodialysis system (1) chest, aligned with and spaced from the upper end of a rear protective door (5), connected to the inside of the internal compartment (A) of the hemodialysis system (1) and to the reservoir (3C) via the tube (3L), and comprising a threaded cap (4A) arranged symmetrically around the entire outer perimeter of the rear inlet (4), serving as the inlet and outlet for all the artificial blood fluid that supplies the reservoir (3C) of the hemodialysis simulator (1);
- the rear protective door (5) arranged vertically, parallel to, and symmetrically centered on the rear of the hemodialysis system (1) chest, fully aligning with the opening of the internal compartment (A) of the hemodialysis simulator (1), comprising a plate (5A) arranged vertically, parallel to, and symmetrically along the entire length of the opening of the internal compartment (A); a set of fixed fasteners (5B) arranged symmetrically spaced around the entire length of the rear of the hemodialysis simulator (1) chest, adjacent to the rear protective door (5) and securing the structure of the rear protective door (5); and a set of removable fasteners (5C) arranged symmetrically spaced around the entire length of the lateral edges of the rear protective door (5) and securing/locking the rear protective door to the hemodialysis simulator (1); and
- a flow control drive circuit (6) arranged symmetrically within the cranial region of the hemodialysis simulator (1) and connected with the microcontroller-based activation circuit of the suction pump (2), and comprising an integrated circuit microcontrollers (6A) featuring Power (J1), ground (J2) and voltage input (J3); a DC power supply battery; and an internal pump (6B) connected to the integrated circuit with microcontrollers (6A) and to the reservoir (3C), operating simultaneously with the water vacuum pump (2B).

2. "HUMANOID HEMODIALYSIS SIMULATOR", according to claim 1 and **characterized by** comprising an electrocardiogram (ECG) simulator located inside the hemodialysis simulator and connected to the flow control drive circuit.

3. "HUMANOID HEMODIALYSIS SIMULATOR", according to claim 1 and **characterized by** a structure whose form resembles that of a pet rather than a humanoid.

4. "HUMANOID HEMODIALYSIS SIMULATOR", according to claim 1 and **characterized by** a structure in which the lower limbs are made of foam, making the legs flexible and bendable.
